# EUROPEAN PATENT APPLICATION

(11) **EP 2 857 550 A1**
(43) Date of publication of application: **08.04.2015**
(21) Application number: 13187091.7
(22) Date of filing: 02.10.2013
(51) Int. Cl.: C23C 16/26, C01B 31/04

(54) **Amine precursors for depositing graphene**

(71) Applicant: BASF SE, 67056 Ludwigshafen (DE); Max-Planck-Gesellschaft zur Förderung der Wissenschaften e.V., 80539 München (DE)
(72) Inventor: Schwab, Matthias Georg, 68199 Mannheim (DE); Müllen, Klaus, 50939 Köln (DE); Sachdev, Herrmann, 66123 Saarbrücken (DE); Ito, Yoshikazu, 982-0826 Sendai, Miyagi (JP)

(57) **Abstract**

The present invention relates to the use of an amine precursor of formula I

(X¹-R¹)ₙ-NH₍₃₋ₙ₎ (I)

or its ammonium salts for depositing a graphene film having a nitrogen content of from 0 to 65 % by weight on a substrate S1 by chemical vapor deposition (CVD),
wherein
R¹
is selected from
(a)
C₁ to C₁₀ alkanediyl, which may all optionally be interrupted by at least one of O, N H and NR²,
(b)
alkenediyl, which may all optionally be interrupted by at least one of O, NH and NR²,
(c)
alkynediyl, which may all optionally be interrupted by at least one of O, NH and NR²,
(d)
C₆ to C₂₀ aromatic divalent moiety, and
(e)
CO and CH₂CO,
X¹
is selected from H, OH, OR², NH₂, NHR², or NR²₂, wherein two groups X¹ may together form a bivalent group X² being selected from a chemical bond, O, NH, or NR²,
R²
is selected from C₁ to C₁₀ alkyl and a C₆ to C₂₀ aromatic moiety which may optionally be substituted by one or more substituents X¹,
n
is 1, 2, or 3.

## Description

The invention relates to the use of amine precursors for depositing graphene and a process of depositing graphene using such amine precursors via chemical vapor deposition (CVD).

### Background of the invention

Graphene ideally consists of sp²-hybridized carbon atoms arranged in a two-dimensional layer and hexagonal array and is the constituting building block of macroscale graphite, with long-range π-conjugation, which results in extraordinary thermal, mechanical, and electronic properties. For manipulating the physical and chemical properties of graphene materials, chemical functionalization is of great interest.

In reality graphene can exhibit intrinsic structural defects, e.g. 5,7,8 membered ring structures, as well as heteroatoms such as boron, nitrogen, oxygen and others, which influence its quality. Doping of graphene means that carbon atoms of the regular graphene lattice can be replaced by those heteroatoms, which may be uncharged or charged or saturated with other functional groups.

In principle, graphene materials can be chemically functionalized. According to a first approach, the aromatic basal plane is modified by addition reaction with C=C bonds. At present, this is the commonly favored approach. Alternatively, chemical functionalization can be effected at the edge of the graphene material, thereby resulting in edge-functionalized graphene (e.g. substituting the edge-bonded residues by another chemical group). This approach is of particular relevance for those graphene materials which have confined dimensions either in just one direction of the plane (graphene nanoribbons) or in both directions of the plane (graphene molecules, i.e. very large polycyclic aromatic compounds).

Graphene exhibits a semi-metal characteristic. The semi-metal characteristic is due to a conduction band and a valance band overlapping each other at only one point (Dirac point). Furthermore, graphene has two-dimensional ballistic transport characteristic. Thus, a mobility of electrons in graphene is generally very high. Because graphene is a zero-gap semiconductor, a field-effect transistor, in which graphene is used as a channel exhibits a very large off-current and a very small on-off ratio. Thus, it is difficult to apply graphene to a field effect transistor. To form a band gap in graphene, it is necessary to break the sublattice symmetry of graphene. In this regard, boron (B) or nitrogen (N) doped graphene, a two dimensional material, is discussed as a promising system with tunable electronic properties. Theory as well as experimental studies indicate that B- or N-doped graphene reveals p- or n-type semiconductor characteristics accompanied by a band gap opening and therefore, graphene with controllable electronic properties is expected to be a promising material for the low cost and eco-friendly replacement of current electronic devices. Furthermore, transparent and doped graphene films are considered for new applications in organic electronic devices such as OLED's and organic solar cells and photodetectors.

So far, few methods for generating N-doped graphene are described, such as thermal or plasma treatments of graphitic materials e.g. by thermal CVD of methane and ammonia gas (Wei, D.; Liu, Y.; Wang, Y.; Zhang, H.; Huang, L.; Yu, G. Nano Lett. 2009, 9, 1752) or molecular precursors like pyridine (Jin, Z.; Yao, J.; Kittrell, C.; Tour, J. M. ACS Nano 2011, 5, 4112) or acetonitrile (Reddy, A. L. M.; Srivastava, A.; Gowda, S. R.; Gullapalli, H.; Dubey, M.; Ajayan, P. M. ACS Nano 2010, 4, 6337-6342) or 1,3,5-triazine (Lu, Y.-F.; Lo, S.-T.; Lin, J.-C.; Zhang, W.; Lu, J.-Y., Liu, F.-H. ACS Nano 2013, 7, 6522-6532) on a Cu substrate or by exposing graphene to a nitrogen (Wang, Y.; Shao, Y.; Matson, D. W.; Li, J.; Lin, Y. ACS Nano 2010, 4, 1790-1798) or ammonia (Lin, Y. C.; Lin, C. Y.; Chiu, P. W. Appl. Phys. Lett. 2010, 96, 133110) containing plasma discharge.

L. Qu, Y. Liu, J.-B. Baek, L. Dai, ACS Nano 2010, 4, 1321 describe the growth of nitrogen doped graphene on sputter-coated Ni substrates by chemical vapor deposition from reactive gas mixtures containing ammonia.

US 20110313194 A1 discloses a graphene comprising a structure of carbon (C) atoms partially substituted with boron (B) atoms and nitrogen (N) atoms, where the graphene has a band gap. Preparation of the graphene comprises performing a chemical vapor deposition (CVD) method using N₂ or NH₃ as an N precursor, BCl₃ as a B precursor and borazine or ammonia borane as a B-N precursor. C₂H₄ and CH₄ are explicitly mentioned as C precursors.

CN 102605339 A discloses process for preparing nitrogen-doped graphene comprising placing a metal catalyst in a reactor, heating the catalyst at 200-600 degrees C under non-oxidizing atmosphere, inletting carbon and nitrogen source in the reactor to react, and processing chemical vapor deposition to obtain the nitrogen-doped graphene. The nitrogen source comprises pyridine, pyrrole, pyrazine, pyridazine, pyrimidine, cytosine, uracil, thymine or purine, and the carbon source comprises methanol, ethanol, benzene, methylbenzene and chlorobenzene.

CN 102887498 A discloses a preparation process of nitrogen-doped graphene by chemical vapor deposition, followed by a doping process. The method includes providing a substrate (Cu, Fe and/or Ni foil) and solid and/or liquid organic carbon source compound (ferrocene, cobaltocene, nickelocene and/or bis- (cyclopentadienyl)manganese), prepaging 5-30 % by weight solution or suspension of the organic carbon source, coating the carbon source solution or suspension to the surface of the substrate, heating the coated substrate to 500-1300 °C under oxygen-free or vacuum condition. After the deposition of the graphene film a gaseous N source compound (N₂, NH₃ and/or methyl amine) is aerated by a flow of 10-200 cm³/s and a C/N molar ratio of 2-20: 1 for reaction to give N-doped graphene, and purifying the obtained N-doped graphene by soaking in dil. acid soln. for 0.1-24h.

It is an object of the present invention to provide a cost efficient and simple process for depositing graphene materials which may optionally be N-doped. It is a further object of the present invention to provide a process for depositing optionally N-doped Graphene. It is yet another object of the present invention to enable a more facile graphene formation by gas phase activation. It is yet another object of the present invention to enable direct graphene formation without the use of additional hydrogen. It is yet another object of the invention to provide a process for directly depositing doped graphene without any additional doping step. It is yet another object of the present invention to prepare a graphene having a better sheet resistance.

### Summary of the invention

One aspect of the present invention is the use of an amine precursor of formula I

(X¹-R¹)ₙ-NH₍₃₋ₙ₎ (I)

or its ammonium salts for depositing a graphene film having a nitrogen content of from 0 to 65 % by weight on a substrate S1 by chemical vapor deposition (CVD),
wherein
- R¹: is selected from
(a) C₁ to C₁₀ alkanediyl, which may all optionally be interrupted by at least one of O, NH and NR₂,
(b) alkenediyl, which may all optionally be interrupted by at least one of O, NH and NR²,
(c) alkynediyl, which may all optionally be interrupted by at least one of O, NH and NR²,
(d) C₆ to C₂₀ aromatic divalent moiety, and
(e) CO and CH₂CO,
- X¹: is selected from H, OH, OR², NH₂, NHR², or NR²₂, wherein two groups X¹ may together form a bivalent group X² being selected from a chemical bond, O, NH, or NR²,
- R²: is selected from C₁ to C₁₀ alkyl and a C₆ to C₂₀ aromatic moiety which may optionally be substituted by one or more substituents X¹,
- n: is 1, 2, or 3.

A further aspect of the present invention is a process for depositing graphene having a nitrogen contend of from 0 to 65 % by weight on a substrate S1, the process comprising
(a) providing an amine precursor of formula I or its ammonium salt as described herein,
(b) providing the substrate S1,
(c) if the amine precursor is in the solid or liquid state, at least partly transferring the amine precursor into the gas phase,
(d) activating the amine precursor in order to decompose the amine precursor, and
(e) depositing the graphene film on the substrate S1.

By using the amine precursors according to the present invention the gas phase activation needed for the graphene formation can be significantly lowered.

Furthermore the amine precursors enable a better and more facile graphene formation, e.g. in the case of methylamine, separation into methyl and amine radicals. Without to be bound to any theory, inventors believe that the amine precursors generate carbon growth species and radical species, which both lead to a modified growth kinetic.

The process according to the present invention enables a direct graphene formation without additional hydrogen, furthermore it also allows the direct manufacture of N-containing or N-doped graphene without the need of any further doping step, and in addition also enables a co-doping with nitrogen and oxygen. The graphene deposited by using the amine precursors according to the present invention show a better sheet resistance.

### Details of the invention

The amine precursors according to formula I

(X¹-R¹)ₙ-NH₍₃₋ₙ₎ (I)

as described herein are useful for depositing a graphene film having a nitrogen content of from 0 to 65 % by weight on a substrate S1 by chemical vapor deposition (CVD). Alternatively its ammonium salts [(X¹-R¹)ₙ-NH₍₄₋ₙ₎]⁺1/m Y^{m-}, with Y being an inorganic or organic, preferably an inorganic counter ion, and m being an integer from 1 to 3, may be used.

R¹ is selected from C₁ to C₁₀ alkanediyl, alkenediyl, and alkynediyl, which may all optionally be interrupted by at least one of O, NH and NR², a C₆ to C₂₀ aromatic divalent moiety and CO or CH₂CO; X¹ is selected from H, OH, OR², NH₂, NHR², or NR²_{2;} R² is selected from H, C₁ to C₁₀ alkyl and a C₆ to C₂₀ aromatic moiety which may optionally be substituted by one or more substituents X¹, wherein two groups X¹ may together form a bivalent group X² being selected from a chemical bond, O, NH, or NR²; and n is 1, 2 or 3.

As used herein, "aromatic moiety" means (i) aryl, such as but not limited to phenyl or naphthyl, (ii) arylalkyl, such as but not limited to toluyl or xylyl, , or (iii) alkylaryl, such as but not limited to benzyl. In a preferred embodiment aromatic moieties are selected from phenyl, benzyl and naphthyl.

As used herein "alkyl", "alkenyl" and "alkynyl" means any monovalent straight chain or cyclic, linear or branched radical derived from the respective alkane, alkene or alkyne, respectively, which may optionally be interrupted by O or NH.

The amine precursors may be primary amines (n=1), secondary amines (n=2) or tertiary amines (n=3). Preferred are primary amines. Ammonium salts of the amine precursors may also be used.

Preferably R¹ is selected from C₁ to C₁₀ alkanediyl, which may optionally be interrupted by O or NH. More preferably R¹ is selected from a linear or branched C₂ to C₅ alkanediyl, which may optionally be interrupted by O atoms, more preferably from C₂ to C₃ alkanediyl, most preferably ethanediyl.

In a particular embodiment the precursors are unsubstituted amines. Preferably X¹ is H and R¹ is selected from linear or branched C₁ to C₅ alkanediyl and a divalent C₆ to C₁₂ aromatic moiety, which means that X¹-R¹ is selected from linear or branched C₁ to C₅ alkyl and a monovalent C₆ to C₁₂ aromatic moiety.

In another embodiment X¹ is selected from NH₂, NHR² or NR²₂ with R² being selected from linear or branched C₁ to C₅ alkyl.

In another embodiment the amine precursor is selected from cyclic amines, such as but not limited to piperidine, piperazine, and morpholine.

In another embodiment the amine precursor is formamide or acetamide.

In yet another embodiment the precursors are alkanolamines or ether derivatives thereof. Preferably X¹ is selected from OH or OR² with R² being selected from linear or branched C₁ to C₅ alkyl, more preferably from C₂ to C₃ alkyl, most preferably ethyl.

Most preferably the amine precursor is selected from methylamine, ethylamine, ethanol amine, methyldiamine, ethylenediamine, aniline, and. combinations thereof. Such amine precursors may also be used in admixture with ammonia.

The graphene film may be deposited on the substrate S1 by using the following process steps:
(a) providing an amine precursor of formula I or its ammonium salt as describe herein,
(b) providing the substrate S1,
(c) if the amine precursor is in the solid or liquid state, at least partly transferring the amine precursor into the gas phase,
(d) activating the amine precursor in order to decompose the amine precursor, and
(e) depositing the graphene film on the substrate S1.

After step (e) a further step (f) may be performed in order to transfer the graphene film to a further substrate S2 as described below.

The activation may be before the deposition on the substrate S1, after the deposition of the amine precursor on the substrate S1 or partly before or after the deposition of the amine precursor on the substrate S1.

The precursor may be used pure, diluted with an inert liquid (NH₃ liquid) or inert gas (He, Ar, N₂) or together with reactive gases (such as H₂, CO, CO₂) or higher Hydrocarbons (C₂H₆, C₂H₄, C₂H₂, etc.) or combined with other dopants such as B₂H₆, BF₃, BCl₃, BBr₃.

It must be emphasized that the amine precursors may be used alone or in combination with other known precursors. It is preferred to use only the amine precursors according to the present invention

The amine precursors according to step (a) are generally available on large scale on the market or may be produced by using standard operations.

Any substrate S1 which is suitable for the growth of graphene and compatible with the deposited graphene can be used in step (b). Preferably, the substrate S1 should also be a material which is compatible with the intended final use. However, as will be discussed below in further detail with regard to a preferred embodiment, it is also possible to provide the graphene film on a substrate S1 first, and transferring the graphene film subsequently to a different substrate S2, which may then become part of the final device.

The substrate S1 of step (b) may generally be selected from an insulating, semiconducting, or conducting substrate or a combination thereof, depending on the application the graphene is used.

The substrate can be chosen from a broad variety of different materials. The substrate can be rigid but may also be flexible (e.g. in the form of a foil). Appropriate substrates include e. g. metals (such as copper, nickel, titanium, platinum and alloys thereof), semiconductors (such as silicon, in particular silicon wafers), inorganic substrates (such as oxides, e.g. SiO₂, glass, HOPG, mica, or any combination thereof), flexible substrates that may be made of e.g. polymers such as polyethylene terephthalate, polyethylene naphthalate, polymethyl methacrylate, polypropylene adipate, polyimide or combinations or blends thereof.

The substrate can be subjected to a pretreatment (such as wet chemical etching, thermal annealing, annealing in a reactive gas atmosphere, plasma cleaning treatment) so as to improve growth of the graphene film and adhesion of the graphene film to the substrate surface.

Step (c) is necessary to perform if the amine precursor is not in the gaseous state of aggregation. In this case the amine precursor needs to be transferred into the gaseous state. This may be done, without limitation, by either direct heating or use of an inert carrier gas and the precursors vapor pressure or by a liquid flow or spray dosage or evaporation system, and also by the use of an inert carrier gas.

In step (d) and (e) the amine precursor is activated and a graphene film is deposited on the substrate by decomposition of the amine precursor.

As used herein "activating" means any conversion of precursors into active species capable of forming graphene, such as but not limited to thermal activation, activation in a plasma or activation by actinic radiation, or combinations thereof. Such activation processes are generally known to a skilled person.

Preferably the activation comprises
(a) thermal activation in the gas phase or on the substrate, or a combination thereof,
(b) plasmachemical gas phase activation,
(c) plasmachemical gas phase activation in combination with a thermal activation on the substrate,
(d) hot filament CVD,
(e) direct laser activation of the precursor(s) by IR, VIS or UV radiation,
(f) UV, VIS and/or IR irradiation of the substrate, the gas phase or both.

The graphene formation can take place either in the gas phase without a substrate (e.g. by plasma CVD), in the gas phase and being then deposited on a substrate, or directly on a substrate.

In step (d) and (e) the surface temperature of the substrate may vary from 4 K to 3000 K depending on the activation method. The substrate may be cooled, heated directly or indirectly by any means of energy transfer, such as thermal, laser, high frequency irradiation, etc.

The gas phase temperature may be equal, higher or lower to the substrate temperature, either be self-defined by the process parameters or separately adjusted. With or without the use of a substrate, the gas phase may be thermally (250 °C - 2600 °C) or plasma-chemically activated with energy densities ranging from 0.001 W/cm³ to 1000 W/cm³

The precursor may be actively evaporated, and evaporated and transported with an inert carrier gas or directly introduced depending on its vapor pressure, boiling point of the precursor, the decomposition temperature, the method of decomposition, and the pressure used.

If thermal decomposition is used, it is preferred to use CVD temperatures of from 500 °C to 1400 °C, most preferably of from 600 °C to 1300 °C. In case of decomposition by plasma activation, energy densities ranging from 0.001 W/cm³ to 1000 W/cm³ may generally be used.

In case of decomposition by actinic radiation a substrate temperatures from 800 °C to 1400 °C, most preferably of from 900 °C to 1200 °C may be used. The actinic radiation may be any radiation capable of breaking chemical bonds, such as but not limited to UV, VIS or IR radiation or a combination thereof.

In step (d) and (e) the total CVD pressure (irrespective of the gas phase composition in which the active precursor(s) may be present from 0.001 % to 100%) may vary from 10⁻⁹ to 500000 hPa depending on the boiling point of the precursor and the temperature used. In one embodiment the pressure is from about 10⁻³ hPa to about 1000 hPa, in particular from about 10-2 hPa to about 500 hPa. In another embodiment the pressure is from about 10⁻⁹ hPa to about 10⁻³ hPa, in particular from about 10⁻⁹ hPa to about 10⁻⁴ hPa. In yet another embodiment the pressure is from about 1100 hPa to about 200 000 hPa, in particular from about 1500 hPa to about 50 000 hPa. In yet another embodiment, atmospheric pressure is used.

One particular advantage of the amine precursors according to the present invention is the possibility to use atmospheric pressure or above. In this way it is much easier to eliminate negative effects by traces of atmospheric oxygen and nitrogen being incorporated in the graphene.

In step (e) a graphene film is deposited on the substrate S1. The graphene may be deposited on the substrate S1 directly or indirectly. In case of indirect deposition the growth may take place in the gas phase leading to any kind of single to multilayer graphene, which is subsequently collected on the substrate S1. Preferred is a direct deposition on the substrate S1.

Generally ideal graphene is an indefinitely large monolayer of carbon atoms arranged in a two-dimensional honeycomb network. "Graphene film" in the terms of the present invention is however not restricted to a material consisting exclusively of single-layer graphene (i.e. graphene in the proper sense and according to the IUPAC definition), but, like in many publications and as used by most commercial providers, rather denotes a material, which is generally a mixture of a single-layer material, a bi-layer material and a material containing 3 to 10 layers and sometimes even up to 100 layers. The individual lateral domains in the plane of the graphene may range from a few nanometers to some mm each. The precise ratio of the different materials (single, bi and multiple layers) depends on the production process. In case of the present invention, the material preferably contains about 0.01 to 99.99% by covered substrate area of single-layer material, the remaining portion being essentially material with other layer composition as specified above.

The graphene film on the substrate consists of individual intergrown domains, which can be single, double or multi layers. The dimension of such domains can be nanoscale up to several mm. In case no substrate is used, the dimension of the individual domains is similar, but the aggregation can be globular or random.

In the terms of the present invention, "doped" relates to hetero (non-carbon) atoms which are incorporated into the graphene lattice, preferably by forming (chemical) bonds between nitrogen and the carbon atoms of the graphene lattice. The nitrogen atoms may be present at the edges as well as at the basal plane of the graphene sheet. However, it is also possible that individual nitrogen atoms are not part of the graphene lattice. Preferably, all or nearly all of the nitrogen atoms provided via the respective starting materials (see below) are incorporated into the graphene lattice in the course of the synthesis reaction. However, it is also possible that smaller amounts of nitrogen atoms be only chemically or physically adsorbed on the surface of the graphene, generally in form of the respective starting material used or of an intermediate formed during the synthesis reaction. Usually, the amount of said chemically or physically adsorbed nitrogen is less than 10 % of the amount of nitrogen forming covalent bonds with the carbon atoms of the graphene lattice.

According to XPS (X-ray photoelectron spectroscopy), the nitrogen-doped graphene according to the invention contains at least part of the nitrogen in form of pyrrolic, pyridinic and/or graphitic nitrogen atoms. Pyridinic nitrogen atoms are part of six-membered rings and are bound to two carbon atoms, thus being part of pyridine-like rings.

Also, hydrogen-containing or charged functions may be present, such as but not limited to-NH₃⁺, -NH₂, -NH₂⁺, NH, NH⁺, etc.. Appropriate counter-ions, such as but not limited to OH-, Cl⁻, SO₄²⁻, PO₄³⁻, or their partially protonated derivatives need to be present.

During synthesis, they may also be oxidized fully or in part, resulting in pyridinic N⁺-O⁻ groups. Graphitic nitrogen atoms are part of six-membered rings and are bound to three carbon atoms, thus being bridging atoms between three fused rings. Such graphitic nitrogen atoms are generally quaternized, suitable counter-ions being single or multiple charged anions or mixtures thereof, for example hydroxide and halides, phosphates, sulfates, carbonates, in particular chloride. While graphitic carbon atoms can be part of a large fused system, pyridinic nitrogen atoms are either on the margin of the system or form "defects" in the honeycomb network.

Without wishing to be bound by theory, it is assumed that the nitrogen-doped graphene according to the invention may comprise the following structural elements (the below structure is to be understood only as a schematic and non-limiting illustration):

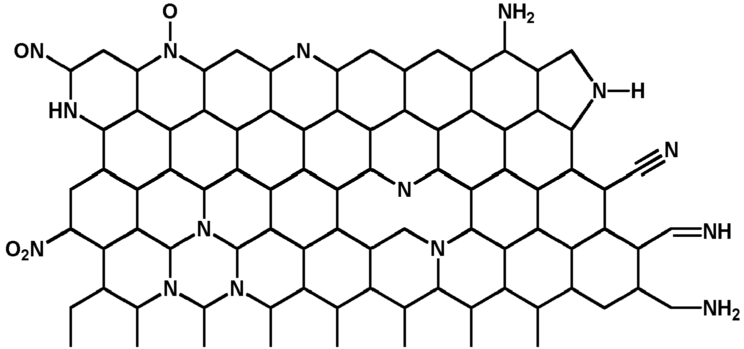

Besides nitrogen (N) further hetero atoms, such as but not limited to oxygen, boron and phosphorus or a combination thereof, may be present in the graphene film. Particularly the co-doping of graphene with nitrogen and boron and/or oxygen is of interest.

The co-doping of nitrogen with oxygen may also enable a chemical functionalization. The co-doping with oxygen may be achieved by either applying a specific N containing precursor and a specified leak, a C-N-O containing precursor, or a C, an N and an O precursor separately.

The deposited graphene film may be free from nitrogen or may contain nitrogen in an amount of from 10⁻²⁰ to 65 % by weight. The term "essentially free from nitrogen" as used herein means that not nitrogen can be detected by using XPS, which is a standard proof to validate the presence of nitrogen. This corresponds to a nitrogen content below 0.3 atom %. Preferably the nitrogen content is below 10⁻² atom %, more preferably below 10⁻⁴ atom %, even more preferably below 10⁻¹⁰ atom %, most preferably below 10⁻²⁰ atom %. If nitrogen is present, the preferred amounts are from the lower limit mentioned before to 20 atom %. In one embodiment amounts of nitrogen in the graphene film up to 0.3 atom % are used. In another embodiment amounts of nitrogen of from 0.3 to 15 atom % are used.

It was found that the nitrogen incorporation into the graphene may be altered by using/not using hydrogen during the CVD process in step (c). The type of nitrogen species may be altered and favored towards the incorporation of nitrogen species with XPS signals in the range of energies lower than 400 eV.

In one particular embodiment the amine precursor is selected from a C₁ to C₄ alkylamine and step (c) is performed in the presence of H₂ in order to deposit a graphene being essentially free from nitrogen.

In another particular embodiment the amine precursor is selected from a C₁ to C₄ alkylamine and step (c) is performed under inert conditions in order to deposit a graphene having a nitrogen content of from 10⁻²⁰ to 65 % by weight.

"Inert conditions" means to use inert gases and conditions which do not constitute to the film. An inert gas may be, but is not limited to, a member of the noble gas family such as e. g. argon.

In yet another particular embodiment the amine precursor is selected from a C₁ to C₄ alkanolamine and step (c) is performed in the presence of H₂ in order to deposit a graphene having a nitrogen content of from 10⁻²⁰ to 65 % by weight.

In yet another particular embodiment the amine precursor is selected from a C₁ to C₄ alkanolamine or methylamine and step (c) is performed under inert conditions in order to deposit a graphene being essentially free from nitrogen.

It was surprisingly found that it is possible to obtain a graphene, which is virtually nitrogen-free and from methylamine as amine precursor.

The CVD of methylamine, ethylamine, ethanol amine, and aniline reveal significant differences compared to the corresponding precursors originating from unsubstituted aliphatic (methane) or aromatic systems (benzene).

The formation of carbon films without significant contribution of high quality graphene is usually indicated by broad D and G modes without any resolved and narrow 2D modes of appropriate line width in the Raman spectra indicating the disordered nature of the films. This was observed for the direct CVD of methane without hydrogen, but also in the case of aromatic precursors like benzene and aniline. This finding might appear surprising since the latter precursors possess six membered-ring structures which formally resemble the graphene lattice. The formation of carbon films from these compounds is assumed to take place via either defragmentation to a manifold of individual gas phase species leading to an uncontrolled nucleation and growth of carbon films, although C-C bond formation between aromatic ring systems may also occur. Graphene films were only formed in the presence of additional hydrogen during the CVD of methane and benzene, whereas e. g. methylamine does give rise to the presence of high quality graphene in the deposits irrespective of the presence of additional hydrogen.

By using the amine precursors according to the present invention a graphene film having a thickness of from 1 to about 100 layers may be deposited. Preferably, the graphene film has a maximum thickness tₘₐₓ of less than 1000 nm, more preferably of less than 100 nm, even more preferably less than 30 nm or even less than 10 nm; and a conductivity σ of at least 100 S/cm, more preferably at least 200 S/cm, even more preferably at least 250 S/cm.

Electrical conductivity is measured by a common four-probe system with a Keithley 2700 Multimeter.

The graphene film can be continuous over an area of at least 1 × 10⁹ µm², more preferably at least 3×10⁸ µm², as determined by optical microscopy at a magnification of 10.

With the term "continuous", it is meant that the substrate surface is completely covered by the graphene film over the area indicated and no substrate surface is detectable within this area by optical microscopy.

As already mentioned above, the process of the present invention also offers the opportunity to prepare the graphene film on the substrate S1 first, and then transfer the graphene film to another substrate S2. Just as an example, the substrate S1 may be made of a material which is more convenient for preparing the graphene film (e.g. high thermal stability, compatible with plasma treatment at high temperature, etc.), whereas the substrate S2 is adapted to the intended use of the final device.

Accordingly, in a preferred embodiment, the process of the present invention comprises a further step (f), wherein the graphene film is transferred to a substrate S2, which is different from the substrate S1.

In principle, any of those materials mentioned above with regard to the substrate S1 can be used for the substrate S2 as well. Of course, a transfer of the graphene film from substrate S1 to substrate S2 is in particular of interest if S2 is different from S1.

For some end applications, it might be of interest to provide the graphene film on a flexible and/or transparent substrate such as flexible and transparent polymer substrates. High flexibility can be achieved by using a very thin substrate which may have a thickness of about 10 to 1000 µm. Furthermore, by selecting appropriate materials such as polymers, a transparent substrate can be provided. A transparent substrate is preferably having a transmittance of at least 50%, more preferably at least 70%, even more at least 90% with regard to a wave length of from 200 to 2000 nm, more preferably 300 to 1000 nm, or 400 to 700 nm.

Thus, in a preferred embodiment, the substrate S2 is a flexible and/or transparent substrate, such as a flexible and/or transparent polymer foil (e.g. a foil made of polyethylene terephthalate, polyethylene naphthalate, polymethyl methacrylate, polypropylene adipate, polyimide or combinations or blends thereof.

The graphene film on the substrate S1 has a lower surface which is in contact with the substrate S1 and an uncovered upper surface. The transfer of the graphene film from the substrate S1 to the substrate S2 can be accomplished by applying the substrate S2 onto the upper surface of the graphene film, followed by removal of the substrate S1 (e.g. by dissolution of the substrate S1 or peeling off the substrate S1).

Alternatively, the transfer can be accomplished by providing a temporary material on the upper surface of the graphene film, followed by removal of the substrate S1 (e.g. by dissolution of the substrate S1 or peeling off the substrate S1) so as to obtain a graphene film now having an uncovered lower surface and an upper surface which is in contact with the temporary material, subsequently applying the substrate S2 onto the lower surface of the graphene film, followed by removal of the temporary material (e.g. by dissolution of the temporary material or peeling off the temporary material) from the upper surface of the graphene film.

Applying the substrate S2 onto the lower surface of the graphene film may include a thermal treatment, so as to improve the adhesion between the substrate S2 and the graphene film.

With the term "temporary material", it is indicated that the material is provided on the graphene film only temporarily and is removed after the graphene film has been attached to the substrate S2.

The temporary material can be a polymer. In the process of the present invention, it is possible that the temporary material such as a polymer is prepared on the upper surface of the graphene film, e.g. by providing a precursor material (such as monomer compounds or an uncured polymer resin) on the upper surface of the graphene film, followed by converting the precursor material into the temporary material (e.g. by polymerization of the monomer compounds or a curing step). Alternatively, it is also possible that the temporary material is prepared externally (i.e. not on the graphene surface) and then provided on the uncovered upper surface of the graphene film. For example a thermal release tape can be applied to the upper surface of the graphene film under. Preferably, such a thermal release tape is applied at mild pressure. A thermal release tape as such is known, e.g. from Bae et al., Nature Nanotechnology, 5, 574-578,2010.

In a preferred embodiment, the temporary material is provided on the upper surface of the graphene film by coating the upper surface with a precursor material, followed by a treatment step (such as polymerization, curing, etc.) so as to convert the precursor material to the temporary material.

In a preferred embodiment which includes such a transfer, a metal (such as copper) is used as substrate S1 and a curable polymer such as polymethyl methacrylate PMMA (i.e. the precursor material) is applied onto the uncovered upper surface of the graphene film, followed by curing the curable polymer so as to provide the temporary material. Subsequently, the metal substrate S1 is removed, e.g. by dissolution in an appropriate etching liquid, from the lower surface of the graphene film. Then, a flexible and optionally transparent polymer foil (e.g. a polyethylene terephthalate foil) is provided on the lower surface of the graphene film, followed by removal of the temporary material, e.g. by dissolution in an appropriate solvent.

After etching, the lower surface of the graphene film can be targeted on the flexible substrate S2. Subsequently, spin-coating can be used to remove the residual water between the graphene film and substrate and increase the interfacial contact. Then, the temporary material (such as PMMA) on the upper surface of the graphene film can be removed and a thermal treatment at about 60-100°C can be carried out.

By using the process as described above, a layered assembly comprising a graphene film on a substrate S1 or S2 may be received.

The graphene films and layered assemblies may be used in manufacturing electronic, optical, or optoelectronic device. Such devices may be, without limitation, a capacitor, an energy storage device, in particular a battery or supercapacitor, an inorganic or organic field effect transistor device, an organic photovoltaic (OPV) device, or an organic light-emitting diode (OLED). Further suitable applications are electrochemical sensors, as well as fuel cells.

The N content in the graphene can be determined by commonly known analytical methods, such as ¹H-NMR spectroscopy, ¹³C-NMR spectroscopy, XPS (X-ray photoelectron spectroscopy), IR spectroscopy and/or mass spectroscopy (e.g. matrix-assisted laser desorption/ionization time of flight (MALDI-TOF) mass spectroscopy).

According to a further aspect, the present invention provides an electronic, optical, or optoelectronic device which comprises a semiconductor film (e.g. a thin film) comprising one or more of the graphene materials as described above.

Preferably, the device is an organic field effect transistor device, an organic photovoltaic device, or an organic light-emitting diode.

Customary and known equipment customarily used in the semiconductor industry can be used for carrying out the process of the invention.

All percent, ppm or comparable values refer to the weight with respect to the total weight of the respective composition except where otherwise indicated. The documents cited in the present application are incorporated herein by reference in their entirety.

The invention will now be described in further detail by the following Examples without restricting the invention thereto.

### Examples

### Comparative Example C1

The graphene films were prepared in a hot wall CVD setup as described in J. Am. Chem. Soc., 2011, 133, 2816-2819, modified with a precursor inlet system allowing the supply of volatile compounds.

Cu foils (7 cm x 7 cm or smaller) were placed in a hot wall furnace inside a quartz tube. The system was evacuated and the leak rate was tested (leak rate: below 10⁻³ hPa/s). The system was flushed with hydrogen gas by maintaining a pressure of 1.5 hPa for cleaning the copper substrate. The tube was heated to 1000 °C (hydrogen flow 150 cm³/min (NTP) at 1.5 hPa). After reaching the desired temperature, 0.20 hPa partial pressure of methane was allowed to react for 20 min at 1.5 hPa, respectively, with or without additional hydrogen flow. After the exposure, the furnace was allowed to cool to room temperature.

The resulting CVD samples on Cu foils were coated with poly methyl methacrylate (PMMA) and then floated in dilute Fe(NO₃)₃ (0.05 g/ml). After dissolution of Cu, the PMMA-coated film was transferred onto a quartz substrate. The PMMA on the film was removed with acetone and the remaining graphene film on quartz was washed with isopropyl alcohol.

The film morphology and the elemental composition of the deposited graphene film were characterized by scanning electron microscopy (SEM), transmission electron microscopy (TEM) and X-ray photoelectron spectroscopy (XPS). The characteristics of the resulting graphene layer are shown in Table 1 and 2.

### Example 2

Comparative example C1 was repeated with 3.0 hPa partial pressure of methylamine. The characteristics of the resulting graphene layer are shown in table 1.

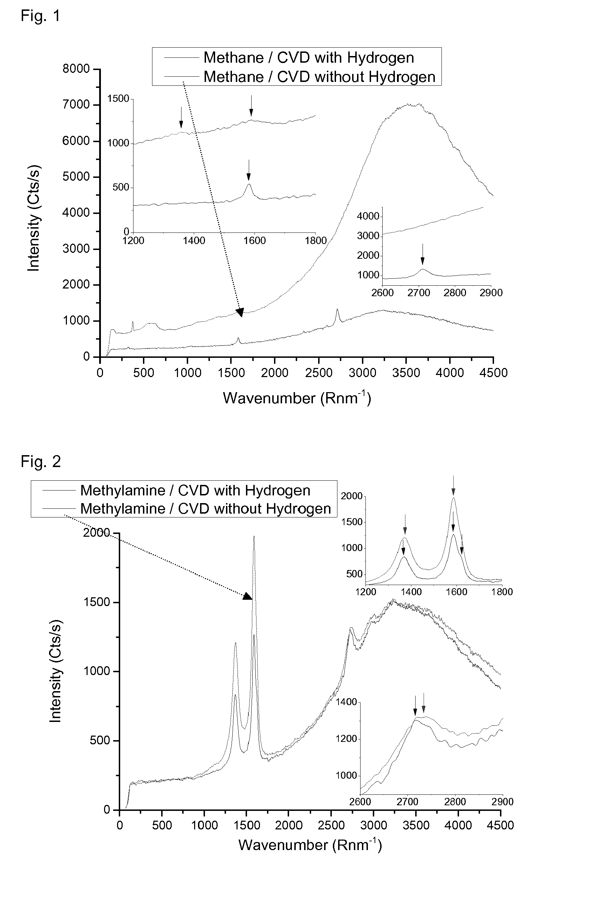

Fig. 2 shows Raman spectra indicating the formation of unstructured carbon or high quality graphene in the case of methane (example C1) and direct formation of graphene in the case of methylamine with additional hydrogen present or absent in the gas phase. This clearly reveals the precursor influence and the role of hydrogen on the CVD of (doped) graphene films.

### Example 3

Comparative example C1 was repeated with 3.0 hPa partial pressure of ethylamine. The characteristics of the resulting graphene layer are shown in table 1. The sheet resistance of the deposited graphene was 3.2 10⁴ Ω/sq.

### Example 4

Comparative example C1 was repeated with 3.0 hPa partial pressure of ethanol amine. The characteristics of the resulting graphene layer are shown in table 1. The sheet resistance of the deposited graphene was 1.9 10⁴ Ω/sq.

### Example 5

Comparative example C1 was repeated with 0.20 hPa partial pressure of aniline. The characteristics of the resulting graphene layer are shown in table 1.

### Comparative Example C6

Comparative example C1 was repeated with 1.0 hPa partial pressure of benzene. The characteristics of the resulting graphene layer are shown in table 1.

**Table 1: Peak positions and line widths (in cm-1) of the Raman D, G and 2 D bands, intensity ratios of D and G peaks for (N-doped) carbon and graphene films grown on copper with or without the presence of hydrogen.**

| Ex. | Precursor | D band Line width | G band Line width | 2D band Line width | I2D/IG |
|---|---|---|---|---|---|
| C1 | Methane | -/1355 | 1584/1588 | 2714/- | 3.7/- |
| | | -/60 | 14/50 | 20/- | |
| 2 | Methyl amine | 1370/1372 | 1588/1588 | 2720/2729 | 0.58/0.36 |
| | | 33/46 | 25/29 | 40/55 | |
| 3 | Ethyl amine | 1376/1376 | 1592.5/1592 | 2741/2741 | 0.90/0.48 |
| | | 40/60 | 20/30 | 41/55 | |
| 4 | Ethanol amine | 1367.5/1366 | 1585.5/1593 | 2729.5/2720 | 1.2/0.67 |
| | | 32/34 | 19/23 | 52/38 | |
| 5 | Aniline | 1370/1378 | 1587/1600 | 2731/- | 0.78/- |
| | | 70/150 | 18/70 | 32/- | |
| C6 | Benzene | 1354/1378 | 1585/1602 | 2700/- | 3.6/- |
| | | 20/150 | 15/60 | 19/ | |

In general, the presence of the 2D band line width is in the range of 40 cm⁻¹ or smaller together with the narrow G band and linewidth of 30 cm-1 or smaller indicates the formation of graphene layers. Single layer graphene reveals a narrow G band with line width lower than 30 cm⁻¹ (usually in the range lower than 20 cm⁻¹) and a high intensity of the 2D band (I 2D > I G) which can be fitted by a single Lorentzian model. If other forms of graphene (e.g. multilayer graphene) and carbon are present (e.g. defective graphene), the intensity of the G and 2 D bands change and the latter decreases. Although D modes are present in the samples indicating defective and disordered carbon at 1350-1370 cm⁻¹, the presence of graphene layers is unambiguously proven by the presence of the narrow 2 D modes at approx. 2700 cm⁻¹ and narrow line width. The Raman analysis of G mode around 1580 cm⁻¹ and a line width better than 30 cm⁻¹ in combination with a 2D mode at 2700 cm⁻¹ and a linewidth better than 40 cm⁻¹ indicates the formation of graphene. N and O content of the deposited graphene was determined by XPS analysis. Both N and O- doping effects can also lead to the presence of D, and D'modes in addition to the G and 2D modes in the Raman spectra.

**Table 2: XPS summary of the C/O/N- ration of the film surfaces:**

| Example | Precursor | Conditions | %C | %O | %N |
|---|---|---|---|---|---|
| C1 | Methane | no H₂ | 59.54 | 39.86 | n/a |
| | CH₄ | with H₂ | 45.04 | 54.96 | n/a |
| 2 | Methylamine | no H₂ | 71.60 | 28.40 | <0.3 |
| | CH₃-NH₂ | with H₂ | 78.10 | 21.90 | <0.3 |
| 3 | Ethylamine | no H₂ | 78.08 | 20.94 | 0.98 |
| | CH₃-CH₂-NH₂ | with H₂ | 73.66 | 26.34 | <0.3 |
| 4 | Ethanolamine | no H₂ | 78.08 | 21.92 | <0.3 |
| | HO-CH₂-CH₂-NH₂ | with H₂ | 56.44 | 42.86 | 0.70 |
| 5 | Aniline | no H₂ | 89.49 | 8.44 | 2.07 |
| | C₆H₅-NH₂ | with H₂ | 89.44 | 9,57 | 1.00 |
| C6 | Benzene | no H₂ | 86.63 | 13.16 | n/a |
| | C₆H₆ | with H₂ | 76.93 | 22.92 | n/a |

Due to the natural leak rate and samples exposed to air after treatment oxygen was incorporated into the film.

The following analytical methods were used:

Chemicals were purchased from commercial suppliers: methane (99.95%), pyridine (99.8%), acetonitrile (99.5%), ethylamine (lecture bottle, 97%), methylamine (97%), nitro methane (98.5%), nitrobenzene (99% extra pure), aniline (99%), benzene (99.5%) from Sigma-Aldrich; nitro ethane (99%), Cu foils (25 µm thickness, 99.8%) from Alfa Aesar; ethanol amine (99%) from ROTH. Liquid precursors were dried and purified by standard distillation techniques prior to use.

### Determination of the film morphology and elemental analysis:

Scanning electron microscopy (SEM) was performed with a Zeiss LEO 1530 Gemini at 1.0 keV and Hitachi SU8000 at 1.0 keV. The transmission electron microscopy (TEM) characterization was done using FEI Tecnai F20. X-ray photoelectron spectroscopy (XPS) was performed using a non-monochromatic Al Kα photon source (1486.6 eV) and a SPECS Phoibos 100 hemispherical energy analyzer. Graphitic carbon materials deposited on a Cu foil were fixed on a sample holder and introduced into the analysis chamber of a custom made ultrahigh vacuum setup (base pressure at 10-10 mbar). The atomic sensitivity factors of the core levels were provided by SPECS. Raman spectra were measured with a BRUKER SENTERRA Spectrometer (488 nm, 2 mW, 200 ms accumulation time, 50 µm aperture, the spectra were analyzed with a Lorentzian fitting). The sheet resistance of transferred graphene films was measured by with a JANDEL micro positioning probe. The transparency of the graphene films on glass substrates was measured with a PERKIN ELMER Lambda 900 UV/VIS/NIR spectrometer.

### Brief description of the drawings:

- Fig. 1: shows a Raman spectrum of the graphene prepared from methane according to comparative example C1.
- Fig. 2: shows a Raman spectrum of the graphene prepared from methylamine according to example 2.

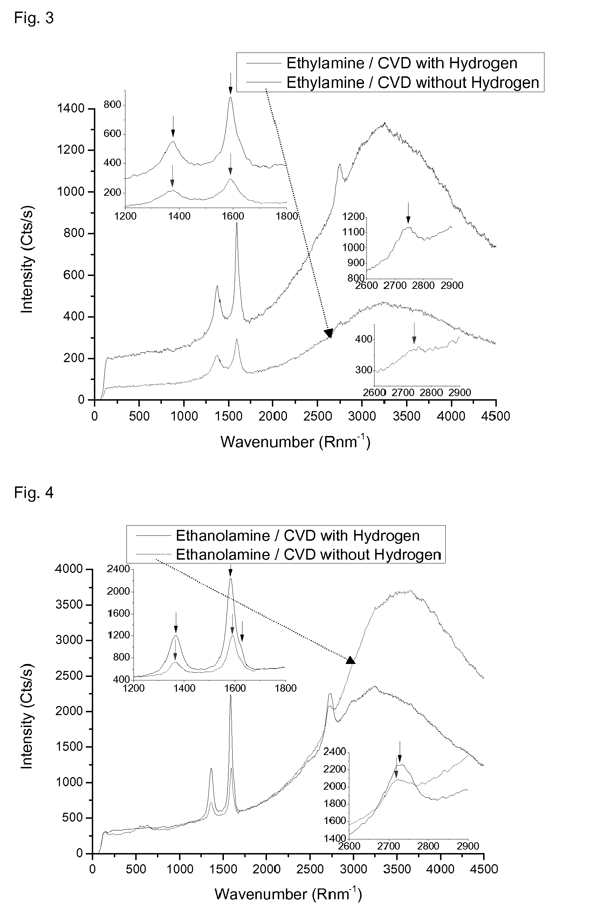

- Fig. 3: shows a Raman spectrum of the graphene prepared from ethylamine according to example 3
- Fig. 4: shows a Raman spectrum of the graphene prepared from ethanolamine according to example 4

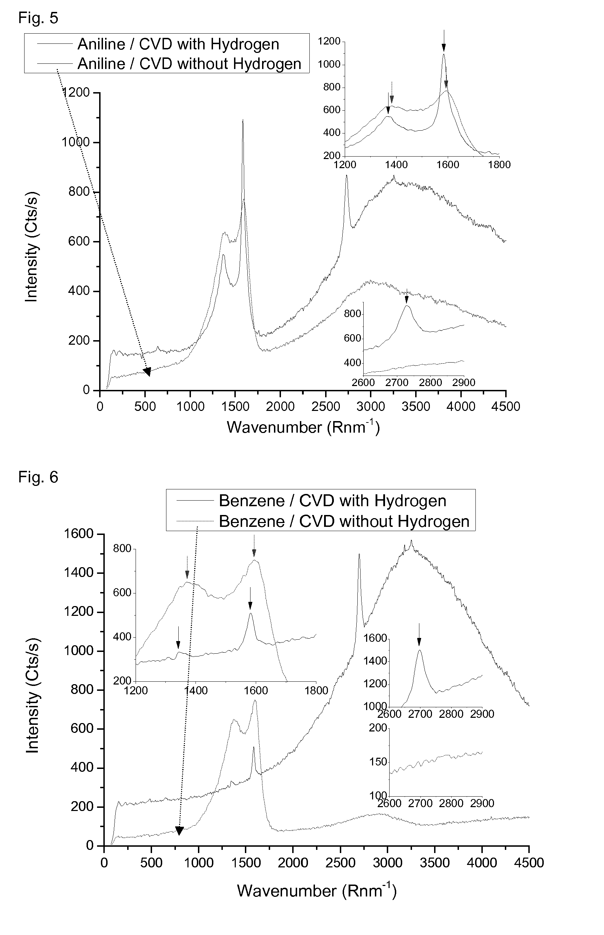

- Fig. 5: shows a Raman spectrum of the graphene prepared from aniline according to example 5
- Fig. 6: shows a Raman spectrum of the graphene prepared from aniline according to comparative example C6

The following embodiments are particularly preferred:
1. Use of an amine precursor of formula I

   (X¹-R¹)ₙ-NH₍₃₋ₙ₎ (I)

   or its ammonium salt for depositing a graphene film having a nitrogen content of from 0 to 65 % by weight on a substrate S1 by chemical vapor deposition (CVD),
   wherein
   - R¹: is selected from
   (a) C₁ to C₁₀ alkanediyl, which may all optionally be interrupted by at least one of O, NH and NR2,
   (b) alkenediyl, which may all optionally be interrupted by at least one of O, NH and NR₂,
   (c) alkynediyl, which may all optionally be interrupted by at least one of O, NH and NR₂,
   (d) C₆ to C₂₀ aromatic divalent moiety, and
   (e) CO, CH₂CO,
   - X¹: is selected from H, OH, OR², NH₂, NHR², or NR²₂, wherein two groups X¹ may together form a bivalent group X² being selected from a chemical bond, O, NH, or NR²,
   - R²: is selected from C₁ to C₁₀ alkyl and a C₆ to C₂₀ aromatic moiety which may optionally be substituted by one or more substituents X¹,
   - n: is 1, 2, or 3.
2. The use according to embodiment 1, wherein R¹ is selected from linear or branched C₁ to C₁₀ alkyl, which may optionally be interrupted by O or NH, and a divalent C₆ to C₁₂ aromatic moiety.
3. The use according to embodiment 1 or 2, wherein X¹ is H and R¹ is selected from linear or branched C₁ to C₅ alkanediyl and a divalent C₆ to C₁₂ aromatic moiety
4. The use according to embodiment 1 or 2, wherein X¹ is selected from OH or OR² with R² being selected from linear or branched C₁ to C₅ alkyl.
5. The use according to embodiment 1 or 2, wherein X¹ is selected from NH₂, NHR² or NR²₂ with R² being selected from linear or branched C₁ to C₅ alkyl.
6. The use according to embodiment 1, wherein the amine precursor is selected from methylamine, ethylamine, ethanol amine, methyldiamine, ethylenediamine, aniline, and combinations thereof.
7. The use according to embodiment 1, wherein the amine precursor is selected from formamide and acetamide, or combinations thereof
8. The use according to anyone of the preceding embodiments, wherein the substrate S1 is selected from an insulating, semiconducting or conducting substrate, or a combination thereof, preferably a metal substrate.
9. A process for depositing a graphene film having a nitrogen content of from 0 to 65 % by weight on a substrate S1, the process comprising
   (a) providing an amine precursor of formula I

      (X¹-R¹)ₙ-NH₍₃₋ₙ₎ (I)

      or its ammonium salt for depositing a graphene film having a nitrogen content of from 0 to 65 % by weight on a substrate S1 by chemical vapor deposition (CVD),
      wherein
      - R¹: is selected from
      (a) C₁ to C₁₀ alkanediyl, which may all optionally be interrupted by at least one of O, NH and NR²,
      (b) alkenediyl, which may all optionally be interrupted by at least one of O, NH and NR²,
      (c) alkynediyl, which may all optionally be interrupted by at least one of O, NH and NR²,
      (d) C₆ to C₂₀ aromatic divalent moiety, and
      (e) CO, CH₂CO,
      - X¹: is selected from H, OH, OR², NH₂, NHR², or NR²₂, wherein two groups X¹ may together form a bivalent group X² being selected from a chemical bond, O, NH, or NR2,
      - R²: is selected from C₁ to C₁₀ alkyl and a C₆ to C₂₀ aromatic moiety which may optionally be substituted by one or more substituents X¹,
      - n: is 1, 2, or 3.
   (b) providing the substrate S1,
   (c) if the amine precursor is in the solid or liquid state, at least partly transferring the amine precursor into the gas phase,
   (d) activating the amine precursor in order to decompose the amine precursor, and
   (e) depositing the graphene film on the substrate S1.
10. The process according to embodiment 9, wherein in step c) amine precursor is heated to a temperature of from 500 °C to 1400 °C, preferably of from 600 °C to 1300 °C, most preferably of from 700 °C to 1200 °C.
11. The process according to embodiments 9 or 10, wherein in step (d) and (e) the pressure is from 10⁻⁹ hPa to 500 000 hPa, preferably from 10⁻⁹ hPa to 2000 hPa.
12. The process according to anyone of embodiments 9 to 11, wherein the amine precursor is selected from a C₁ to C₄ alkylamine and steps (d) and (e) are performed in the presence of H₂ in order to deposit a graphene film being essentially free from nitrogen.
13. The process according to anyone of embodiments 9 to 11, wherein the amine precursor is selected from a C₁ to C₄ alkylamine and steps (d) and (e) are performed under inert conditions in order to deposit a graphene film having a nitrogen content of from 10⁻²⁰ to 65 % by weight.
14. The process according to anyone of embodiments 9 to 11, wherein the amine precursor is selected from a C₁ to C₄ alkanolamine and steps (d) and (e) are performed in the presence of H₂ in order to deposit a graphene film having a nitrogen content of from 10⁻²⁰ to 65 % by weight.
15. The process according to anyone of embodiments 9 to 11, wherein the amine precursor is selected from a C₁ to C₄ alkanolamine or methylamine and steps (d) and (e) are performed under inert conditions in order to deposit a graphene film being essentially free from nitrogen.
16. The process according to anyone of the preceding embodiments, comprising a further step (f) transferring the graphene film from the substrate S1 to a substrate S2, which is different from the substrate S1.
17. A layered assembly comprising a graphene film on a substrate S1 or S2, said layered assembly being obtainable by the process according to one of the embodiments 9 to 16, and said graphene film having a nitrogen content of from 0 to 65 % by weight.
18. An electronic, optical, or optoelectronic device obtainable by a process according to anyone of the embodiments 9 to 16.
19. The device according to embodiment 18, wherein the device is a capacitor, an energy storage device, in particular a battery or supercapacitor or fuel cell, a field effect transistor device, an organic photovoltaic device, or an organic light-emitting diode, a photodetector or a electrochemical sensor.

## Claims

1. Use of an amine precursor of formula I
(X¹-R¹)ₙ-NH₍₃₋ₙ₎ (I)
or its ammonium salt for depositing a graphene film having a nitrogen content of from 0 to 65 % by weight on a substrate S1 by chemical vapor deposition (CVD),
wherein
R¹ is selected from
(a) C₁ to C₁₀ alkanediyl, which may all optionally be interrupted by at least one of O, NH and NR²,
(b) alkenediyl, which may all optionally be interrupted by at least one of O, NH and NR²,
(c) alkynediyl, which may all optionally be interrupted by at least one of O, NH and NR²,
(d) C₆ to C₂₀ aromatic divalent moiety, and
(e) CO, CH₂CO,
X¹ is selected from H, OH, OR², NH₂, NHR², or NR²₂, wherein two groups X¹ may together form a bivalent group X² being selected from a chemical bond, O, NH, or NR²,
R² is selected from C₁ to C₁₀ alkyl and a C₆ to C₂₀ aromatic moiety which may optionally be substituted by one or more substituents X¹,
n is 1, 2, or 3.

2. The use according to claim 1, wherein R¹ is selected from linear or branched C₁ to C₁₀ alkyl, which may optionally be interrupted by O or NH, and a divalent C₆ to C₁₂ aromatic moiety.

3. The use according to claim 1 or 2, wherein X¹ is H and R¹ is selected from linear or branched C₁ to C₅ alkanediyl and a divalent C₆ to C₁₂ aromatic moiety

4. The use according to claim 1 or 2, wherein X¹ is selected from OH or OR² with R² being selected from linear or branched C₁ to C₅ alkyl.

5. The use according to claim 1 or 2, wherein X¹ is selected from NH₂, NHR² or NR²₂ with R² being selected from linear or branched C₁ to C₅ alkyl.

6. The use according to claim 1, wherein the amine precursor is selected from methylamine, ethylamine, ethanol amine, methyldiamine, ethylenediamine, aniline, and combinations thereof.

7. The use according to claim 1, wherein the amine precursor is selected from formamide and acetamide, or combinations thereof

8. The use according to anyone of the preceding claims, wherein the substrate S1 is selected from an insulating, semiconducting or conducting substrate, or a combination thereof, preferably a metal substrate.

9. A process for depositing a graphene film having a nitrogen content of from 0 to 65 % by weight on a substrate S1, the process comprising
(a) providing an amine precursor of formula I
(X¹-R¹)ₙ-NH₍₃₋ₙ₎ (I)
or its ammonium salt for depositing a graphene film having a nitrogen content of from 0 to 65 % by weight on a substrate S1 by chemical vapor deposition (CVD),
wherein
R¹ is selected from
(a) C₁ to C₁₀ alkanediyl, which may all optionally be interrupted by at least one of O, NH and NR²,
(b) alkenediyl, which may all optionally be interrupted by at least one of O, NH and NR₂,
(c) alkynediyl, which may all optionally be interrupted by at least one of O, NH and NR²,
(d) C₆ to C₂₀ aromatic divalent moiety, and
(e) CO, CH₂CO,
X¹ is selected from H, OH, OR², NH₂, NHR², or NR²₂, wherein two groups X¹ may to-gether form a bivalent group X² being selected from a chemical bond, O, NH, or NR²,
R² is selected from C₁ to C₁₀ alkyl and a C₆ to C₂₀ aromatic moiety which may optional-ly be substituted by one or more substituents X¹,
n is 1, 2, or 3.
(b) providing the substrate S1,
(c) if the amine precursor is in the solid or liquid state, at least partly transferring the amine precursor into the gas phase,
(d) activating the amine precursor in order to decompose the amine precursor, and
(e) depositing the graphene film on the substrate S1.

10. The process according to claim 9, wherein the amine precursor is selected from a C₁ to C₄ alkylamine and steps (d) and (e) are performed in the presence of H₂ in order to deposit a graphene film being essentially free from nitrogen.

11. The process according to claims 9, wherein the amine precursor is selected from a C₁ to C₄ alkylamine and steps (d) and (e) are performed under inert conditions in order to deposit a graphene film having a nitrogen content of from 10⁻²⁰ to 65 % by weight.

12. The process according to claim 9, wherein the amine precursor is selected from a C₁ to C₄ alkanolamine and steps (d) and (e) are performed in the presence of H₂ in order to deposit a graphene film having a nitrogen content of from 10⁻²⁰ to 65 % by weight.

13. The process according to anyone of claims 9 to 11, wherein the amine precursor is selected from a C₁ to C₄ alkanolamine or methylamine and steps (d) and (e) are performed under inert conditions in order to deposit a graphene film being essentially free from nitrogen.

14. The process according to anyone of the preceding claims, comprising a further step (f) transferring the graphene film from the substrate S1 to a substrate S2, which is different from the substrate S1.

15. A layered assembly comprising a graphene film on a substrate S1 or S2, said layered assembly being obtainable by the process according to one of the claims 9 to 16, and said graphene film having a nitrogen content of from 0 to 65 % by weight.
